# EUROPEAN PATENT SPECIFICATION

(11) **EP 1 872 796 B1**
(45) Date of publication and mention of the grant of the patent: **21.09.2011**
(21) Application number: 06714545.8
(22) Date of filing: 24.02.2006
(51) Int. Cl.: A61K 45/06, A61K 31/192, A61K 31/196, A61K 31/381, A61K 31/40, A61K 31/403, A61K 31/423, A61K 31/53, A61K 31/5415, A61K 47/08, A61K 47/10, A61K 9/06

(54) **TRANSDERMAL PREPARATION FOR EXTERNAL USE CONTAINING NONSTEROIDAL ANTIINFLAMMATORY/ANALGESIC AGENT**
TRANSDERMALE ZUBEREITUNG ZUR ÄUSSERLICHEN ANWENDUNG MIT EINEM NICHTSTEROIDALEN ENTZÜNDUNGSHEMMER/ANALGETIKUM
PREPARATION TRANSDERMIQUE A USAGE EXTERNE CONTENANT UN AGENT ANALGESIQUE/ANTIINFLAMMATOIRE NON STEROIDIEN

(30) Priority: 25.02.2005 JP 2005050991
(43) Date of publication of application: 02.01.2008
(73) Proprietor: Hisamitsu Pharmaceutical Co., Inc., Tosu-shi, Saga 841-0017 (JP)
(72) Inventor: YOSHITAKE, Kazuhisa c/o Hisamitsu Pharmaceutical Co., Inc., Tosu-shi, Saga, 8410017 (JP); ATARASHI, Kenji c/o Hisamitsu Pharmaceutical Co., Inc., Tsukuba-shi Ibaraki, 3050856 (JP); KUWAHARA, Tetsuji c/o Hisamitsu Pharmaceutical Co., Inc., 25-11 Kannondai 1-chome Tsu kuba-shi, Ibaraki, 3050856 (JP); IKESUE, Koichi c/o Hisamitsu Pharmaceutical Co., Inc., Tosu-shi, Saga, 8410017 (JP); SAKAI, Michinori c/o Hisamitsu Pharmaceutical Co., Inc., Tsukuba-shi, Ibaraki, 3050856 (JP); HASHIMOTO, Yoshiaki c/o Hisamitsu Pharmaceutical Co., Inc., Tosu-shi, Saga, 8410017 (JP); TSURUDA, Kiyomi c/o Hisamitsu Pharmaceutical Co., Inc., Tosu-shi, Saga, 8410017 (JP)
(74) Representative: Müller, Christian Stefan Gerd
(86) International application number: PCT/JP2006/303406
(87) International publication number: WO 2006/090833

(56) References cited:
- WO-A1-01/68061
- WO-A1-96/08245
- WO-A1-2004/098575
- WO-A1-2005/023307
- WO-A1-2005/063215
- WO-A1-2005/123136
- WO-A2-2005/104825
- JP-A- 60 155 111
- DATABASE WPI Week 198539 Thomson Scientific, London, GB; AN 1985-239345 XP002536942 & JP 60 155111 A (HISAMITSU PHARM CO LTD) 15 August 1985 (1985-08-15)
- YAMAZAKI F ET AL: "Non-Steroid-kei Shoen Chintsuzai no Kodokusei, Hikari Kansano Oyobi Jizokusei Kosen Kabin ni Taisuru Kento /EXPERIMENTAL STUDIES ON PHOTOTOXICITY,PHOALLERGENICITY AND PROLONGED PHOTOSENSITIVITY DUE TO NONSTEROIDAL ANTI-INFLAMMATORY DRUGS" NITIKAWA KAISHI, XX, JP, vol. 113, no. 4, 1 January 2002 (2002-01-01), pages 405-411, XP003004572
- KAMIDE R: "DRUG-INDUCED PHOTOSENSITIVITY -AN UPDATE-" PHOTOMEDICINE AND PHOTOBIOLOGY, NAGOYA, JP, vol. 21, 1 January 1999 (1999-01-01), pages 9-10, XP003004573 ISSN: 0912-232X
- RADSCHUWEIT ET AL: "UV-induces formation of hydrogen peroxide based on the photochemistry of ketoprofen" PHOTOCHEMISTRY AND PHOTOBIOLOGY, BLACKWELL PUBLISHING, OXFORD, GB, vol. 73, no. 2, 1 February 2001 (2001-02-01), pages 119-127, XP002985755 ISSN: 0031-8655
- DATABASE WPI Week 200170 Thomson Scientific, London, GB; AN 2001-611377 XP002536943 & WO 01/68061 A (HISAMITSU PHARM CO LTD) 20 September 2001 (2001-09-20)
- DATABASE WPI Week 200516 Thomson Scientific, London, GB; AN 2005-151928 XP002536944 & WO 2004/098575 A (HISAMITSU PHARM CO) 18 November 2004 (2004-11-18)
- IDEMITSU T.: 'Hikari Sesshoku Hifuen' THE ALLERGY IN PRACTICE vol. 15, no. 9, 1995, pages 620 - 623, XP003004571
- YAMAZAKI F. ET AL.: 'Non-Steroid-kei Shoen Chintsuzai no Kodokusei, Hikari Kansano Oyobi Jizokusei Kosen Kabin ni Taisuru Kento' NITIKAWA KAISHI vol. 113, no. 4, 2002, pages 405 - 411, XP003004572
- KAMIDE R.: 'DRUG-INDUCED PHOTOSENSITIVITY -AN UPDATE-' PHOTOMED. PHOTOBIOL. vol. 21, 1999, pages 9 - 10, XP003004573
- RADSCHUWEIT A. ET AL.: 'UV-Induces Formation of Hydrogen Peroxide Based on the Photochemistry of Ketoprofen' PHOTOCHEM. PHOTOBIOL. vol. 73, no. 2, 2001, pages 119 - 127, XP002985755

## Description

### [Technical Field]

The present invention relates to a transdermal preparation for external use which contains a nonsteroidal antiinflammatory drug (NSAID).

### [Background Art]

Since NSAIDs such as ketoprofen have an excellent antiinflammatory and analgesic action, they are contained as an efficacious component in each type of transdermal preparations for external use such as patches including poultices and plasters, gels, creams, ointments and liniments. However, some of transdermal preparations for external use of an NSAID are photosensitive, and it has been reported that there is a possibility to induce the onset of skin symptoms due to non-immunological or immunological mechanisms upon excessive exposure of light, though it is very rare.

As a trial to suppress skin symptoms resulting from a photosensitive drug, an example trying to suppress the formation of photolytes by inhibiting photolysis of ketoprofen while blending a UV absorber to a transdermal preparation for external use which contains ketoprofen(see Patent document 1), an example to apply a UV blocking treatment to a backing of a patch containing an NSAID (see Patent document 2), and an example to blend titanium oxide to an antiinflammatory preparation for external use to the skin (see Patent document 3) have been reported to date. However, a preparation improvement aiming to further inhibit the onset of skin symptoms has been desired.
Patent document 1: JP, A, 60-155111
Patent document 2: WO 01/68061
Patent document 3: JP, A, 9-169658
Patent document 4: JP, A, 53-99316
Patent document 5: JP, A, 56-22711
Patent document 6: JP, A, 2000-136122
Non-patent document 1: Nihon Hifukagakkaizasshi, 113(4), 405-411 (2003)
Non-patent document 2: Photochemistry and photobiology, 73(2), 119-27 (2001)

### [Disclosure of the Invention]

### [Problems to be Solved by the Invention]

The invention makes it an object to further ensure, in a transdermal preparation for external use containing a photosensitive NSAID, the inhibition of skin symptoms resulting from the above-described agent which received irradiation of light.

### [Means to Solve the Problems]

During extensive research to attain the above object, the inventors noticed that as to skin symptoms resulting from a photosensitive drug, there are those occurring due to a non-immunological mechanism in which an active oxygen produced when the drug receives irradiation of sunlight brings damage to tissues/cells and those in which a drug made haptenic by exposure of excessive sunlight brings disorder to tissues/cells via an immunological mechanism; the former is indifferent to disposition of an individual and there is a possibility that it appears by exposure of plenty UV rays, and in the meantime, the latter appears only for a part of individuals sensitized with a haptenic drug in an immunological way and there is a possibility that the drug can not be used when once sensitized, and therefore, it was necessary to suppress the both mechanisms in order to surely inhibit the above-described skin symptoms.

On the basis of information (see Non-patent documents 1 and 2) that skin symptoms resulting from a photosensitive drug are induced by UV rays, in particular, ultraviolet-A (UVA: wave length 320-400nm), the inventors, when carrying out trials to mix various UVA blockers in preparations, noticed that all the UVA blockers were not necessarily effective for inhibition of the above-described skin symptoms and some of them made inversely the symptoms worse. Therefore, as results of further investigation, surprisingly, the inventors found that among UV blockers including UVA blockers and UVB blockers, UV blockers having a high ability to migrate into the skin was particularly effective for suppression of both of the non-immunological and immunological symptoms considered to constitute the above-described skin symptoms, and accomplished the invention. It has not been known at all that the skin migration of a UV blocker gets involved in suppression of the above-described skin symptoms, and therefore, such effects of the invention far exceed expectations of a person skilled in the art.

Namely, the invention relates to a transdermal preparation for external use which contains a photosensitive NSAID and a UV blocker migrating into the skin,
wherein the UV blocker is selected from the group consisting of 4-*tert*-butyl-4'-methoxydibenzoylmethane, n-hexyl 2-(4-diethylamino-2-hydroxybenzoyl)benzoate, 4-hydroxy-3-methoxycinnamic acid, a branched alkyl ester of 4-hydroxy-3-methoxycinnamic acid, terephthalylidene-3,3'-dicamphor-10,10'-disulfonic acid, 2-(2H-benzotriazol-2-yl)-4-methyl-6-[2-methyl-3-[1,3,3,3-tetramethyl-1-[(trimethylsilyl)oxy]disiloxanyl]propyl]phenol, 2-ethylhexyl dimethoxybenzylidenedioxoimidazolidinepropionate, 1-(3,4-dimethoxyphenyl)-4,4-dimethyl-1,3-pentanedione 2-(2'-hydroxy-5'-methoxyphenyl)-benzotriazole, and oxybenzone.
In addition, the invention relates to the transdermal preparation for external use, wherein the NSAID is selected from the group consisting of ketoprofen, tiaprofenic acid, suprofen, tolmetin, carprofen, benoxaprofen, piroxicam, benzydamine, naproxen, diclofenac, ibuprofen, diflunisal, azapropazone, and pharmaceutically acceptable salts thereof.

### [Effect of the Invention]

In a transdermal preparation for external use of the invention, the onset of light-induced skin symptoms by a photosensitive NSAID is remarkably suppressed. Namely, in the invention, production of an active oxygen and a free radical from the photosensitive drug and transformation of said drug into hapten, which are considered reasons of the above described skin symptoms, are effectively suppressed by a UV blocker migrating into the skin.

In a transdermal preparation for external use of the invention, although a mechanism to suppress such skin symptoms are not necessarily clear, the above described symptoms seem to be suppressed effectively by migration of a UV blocker together with a photosensitive NSAID into the skin, and therefore by suppression of a photochemical reaction and the like of said NSAID in the skin as well as in the preparation.
Although a preparation for external use which is blended with a UV absorber has been known (see Patent documents 1, 3-6), these aimed at reduction of a bad influence in a preparation by UV rays (see Patent documents 1, 3-5) and sunburn protection (see Patent document 6), and there was no example which focused on the skin migration of a UV blocker. For example, although a transdermal preparation for external use which contains a UV protector such as 2-ethylhexyl *p*-methoxy-cinnamate, an anti-inflammatory agent such as indomethacin and a metal chelating agent is described in Patent document 6, any example containing a photosensitive NSAID used in the invention is not described; also in the effect of that invention, only non-immunological skin symptoms such as erythema and pigmentation due to a UV exposure are examined and there is no description on immunological skin symptoms; therefore, there is no information on the existence itself of skin symptoms due to an immunological mechanism resulting from the photosensitive NSAID and on a way how to suppress them. Consequently, the transdermal preparation for external use inhibiting skin symptoms due to the photosensitive NSAID by blending a UV blocker migrating into the skin was realized for the first time in the present invention.

The transdermal preparation for external use of the invention can sufficiently inhibit the onset of skin symptoms resulting from the photosensitive drug which are produced by non-immunological or immunological mechanisms and can also exert an antiinflammatory/analgesic effect, and therefore, the application as a medicine which is extremely high in safety can be expected.

### [Best Mode for Carrying out the Invention]

In the following, favorable examples of the transdermal preparation for external use of the invention are explained in more detail.
The transdermal preparation for external use of the invention is characterized in that, it contains a photosensitive NSAID and a UV blocker migrating into the skin.

The UV blocker used in the transdermal preparation for external use of the invention is preferably one which accumulates in the skin and, in particular, in the horny layer. A preferred branched alkyl ester of 4-hydroxy-3-methoxycinnamic acid is isostearyl 4-hydroxy-3-methoxycinnamate.
In the transdermal preparation for external use of the invention, a particularly preferable blocker is 4-*tert*-butyl-4'-methoxydibenzoylmethane.
The skin migration amount (storage amount) of the UV blocker can be determined, for example, by a method wherein a piece of skin taken from a hairless mouse is spread on a filter paper wetted with a physiological solution such as physiological saline, applied with a test substance, and let it stand for a designated amount of time, for example, 4-8 hours, typically 6 hours, and then a designated area of the piece of skin, which makes the part applied with the test substance center, is cut and followed by removal of remaining test substance on the skin surface and then the mass of the test substance in the skin is quantified.

The blend amount of the above blocker in the transdermal preparation for external use of the invention is not particularly limited; however, it is preferably 0.01-20 weight % based on the total amount of the preparation, more preferably 1-10 weight %, particularly preferably 2-5 weight %. When the blend amount of the UV blocker is not less than the above described lower limit, the skin symptoms can effectively suppressed, and on the other hand, when it is not more than the above described upper limit, an undesirable reaction can be avoided. Further, here, the term "suppress" means that by containing the UV blocker, a numerical value concerning skin symptoms due to a non-immunological mechanism evaluated, e.g., by an auricular photo-irradiation test (see Example 1) and a numerical value concerning skin symptoms due to an immunological mechanism evaluated by a local lymph node test (see Examples 2 and 4), and a skin photo-sensitization test (see Example 3) are reduced respectively compared with those of cases in which the above described UV blocker is not contained. The degree of reduction (suppression ratio) is preferably not less than 4%, more preferably not less than 30%, more preferably not less than 50%, most preferably not less than 60%.

As described above, the invention surely inhibits skin symptoms induced by non-immunological or immunological mechanisms in the transdermal preparation for external use which contains a photosensitive NSAID as an efficacious component. As such an NSAID, it is not particularly limited as long as it has photosensitivity, and more typically possibility to induce the above described skin symptoms upon photo-irradiation. Examples include ketoprofen, tiaprofenic acid, suprofen, flurbiprofen, loxoprofen, tolmetin, carprofen, benoxaprofen, piroxicam, benzydamine, naproxen, diclofenac, ibuprofen, diflunisal, azapropazone, felbinac, methyl salicylate, ethyleneglycol salicylate, valdecoxib, celecoxib rofecoxib, acetaminophen, mefenamic acid, clofezone, sulpyrine, aminoprofen, naproxen, pranoprofen, mepirizole, oxaprozin, tenoxicam, lornoxicam, meloxicam and/or pharmaceutically acceptable salts thereof; among them ketoprofen, tiaprofenic acid, suprofen, and tolmetin, which have a skeleton similar to benzophenone in the structure, are preferable, and ketoprofen having a benzophenone skeleton is particularly preferable. Such NSAIDs may be used alone or in a combination of two or more kinds.

The blend amount of the above NSAID in the transdermal preparation for external use of the invention is not particularly limited; however, it is preferably 0.1-10 weight % based on the total amount of the preparation. When the blend amount of the NSAID is not less than the above described lower limit, a favorable antiinflammatory/analgesic effect is obtained.
A preferable combination of the UV blocker and the NSAID includes, but not limited to, a combination of ketoprofen and one or more UV blockers selected from the group consisting of 4-*tert*-butyl-4'-methoxydibenzoylmethane, 2-(2'-hydroxy-5'-methoxyphenyl)-benzotriazole, terephthalylidene-3,3'-dicamphor-10,10'-disulfonic acid, and oxybenzone. Among these, a combination of ketoprofen and one or more UV blockers selected from the group consisting of 4-*tert*-butyl-4'-methoxydibenzoylmethane, terephthalylidene-3,3'-dicamphor-10,10'-disulfonic acid, and oxybenzone is more preferable, and the combination of 4-*tert*-butyl-4'-methoxydibenzoylmethane and ketoprofen is particularly preferable. In the meantime, since 2,2',4,4'-tetrahydroxybenzophenone, 4-aminobenzoic acid, and ethylene glycol monosalicylate, which are UV blockers described in Patent document 6, it is not preferable to combine them with ketoprofen in the present invention, because of the possibility to worse the above described skin symptoms.

In the transdermal preparation for external use of the invention, it is preferable to contain a base of each preparation according to a dosage form of the preparation in addition to the above described essential components (NSAID, UV blocker for the present invention). As dosage forms of the transdermal preparation for external use of the invention, illustrative are patches such as a poultice or a plaster, gels, creams, ointments, liniments and the like. Among these, the patches excellent in absorbability of the NSAID are preferable in view of a favorable onset of effects of the invention. In the following, a base as well as a formulation example are explained according to each dosage form of the transdermal preparation for external use of the invention.

First, a poultice is explained. A poultice base used in the poultice of the invention is not limited, and may be selected among those conventionally used. As components contained in such a poultice base, examples include thickeners (synthetic aqueous polymer such as sodium polyacrylate, polyacrylic acid, polyvinyl alcohol, polyvinyl pyrrolidone, polyethyleneoxide and polyvinyl methacrylate, natural substances such as gum arabic, starch and gelatin, methyl cellulose, hydroxypropyl cellulose, alginic acid, sodium alginate, ammonium alginate, sodium carboxymethyl cellulose, etc.), humectants (urea, glycerin, polyethylene glycol, propylene glycol, butylene glycol, sorbitol, etc.), fillers (kaolin, talc, bentonite, epoxy resins, organic acids (citric acid, tartaric acid, maleic acid, maleic anhydride, succinic acid, etc.), calcium, magnesium, aluminum, etc.), water, solubilizers (propylene carbonate, crotamiton, diisopropyl adipate, etc.), irritation inhibitors (diphenhydramine hydrochloride, chlorpheniramine maleate, glycyrrhizinic acid, dexamethasone, betamethasone, fluocinolone acetonide, etc.), other additives (salicylic acid, methyl salicylate, ethyleneglycol salicylate, 1-menthol, camphor, vanillyl amide nonylate, red pepper extract, peppermint oil, Azone(R), etc.) and the like, and by blending the above described essential components with a poultice base prepared by mixing various components selected among these, the poultices of the invention can be prepared.

Next, one favorable production example (formulation example) of poultices is shown. Namely, first, the above NSAID 0.1-10 parts by weight, the above UV blocker 0.01-20 parts by weight are mixed and dissolved in a solubilizer 0.5-8 parts by weight to obtain homogeneous mixture A. In the meantime, a thickener 5-20 parts by weight (preferably 10-15 parts by weight) is dissolved under mixing and dispersing in a humectant 5-40 parts by weight and water 10-80 parts by weight and further added with a filler not more than 20 parts by weight to obtain homogeneous kneaded product B. Then, the mixture A is added to the kneaded product B to obtain a homogeneous kneaded product. The obtained kneaded product is expansively coated on a backing in a conventional way and followed by sticking a removable cover on it to obtain a poultice of the invention. As the backing, a stretch or non-stretch backing can be used. As such backings, specifically, examples include cloth, non-woven cloth, polyurethane, polyester, polyvinyl acetate, polyvinylidene chloride, polyethylene, polyethylene terephthalate, aluminum sheet, or a composite material thereof and the like. In addition, as to the removable cover, examples include those consisting of polyethylene, polypropylene, polyvinyl chloride, polyester, polyvinylidene chloride and paper treated with silicone, and the like.

Next, a plaster is explained. A plaster base used in the plaster of the invention is not particularly limited and may be selected among those conventionally used. As components contained in such a plaster base, examples include polymer bases (an acrylic composition which is copolymer of vinyl monomers such as methacrylate, acrylonitrile, vinyl acetate or vinyl propionate, a silicone resin, a polyisoprene rubber, a natural rubber, an acrylic rubber, a styrene-butadiene-styrene block copolymer, a styrene-isoprene-styrene block copolymer, etc.), oils or higher fatty acids (almond oil, olive oil, camellia oil, persic oil, peanut oil, olein oil, liquid paraffin, polybutene, etc.), tackifiers (rosin, a maleic acid-modified rosin ester, a hydrogenated rosin ester, etc.), fatty acid metal salts (zinc undecylenate, zinc stearate, calcium stearate, aluminum stearate, magnesium stearate, sodium stearate, zinc palmitate, zinc myristate, magnesium myristate, sodium laurate, zinc laurate, etc.), irritation inhibitors, other additives (salicylic acid, methyl salicylate, ethyleneglycol salicylate, 1-menthol, camphor, vanillyl amide nonylate, red pepper extract, peppermint oil, Azone(R), etc.) and the like, and by blending the above described essential components with a plaster base prepared by mixing various components selected among these, the plasters of the invention can be prepared.

Next, one favorable production example (formulation example) of plasters is shown. Namely, in case of producing by a hot-melt method, first, the above polymer base 5-40 parts by weight, the above oil or fatty acid 20-70 parts by weight, the above tackifier 10-40 parts by weight, and the above fatty acid metal salt 0.1-10 parts by weight are heated and mixed at 120-160°C using a mixing machine such as a kneader, a mixer or the like, then the above NSAID 0.1-10 parts by weight, and the above UV blocker 0.01-20 parts by weight are added and mixed thereto. The obtained mixture may directly be spread on a backing, or once spread on a paper, a film, or the like in which a removable treatment is carried out, and then transferred to a desirable backing in a covered state by compression. On the other hand, in case of producing by a solvent method, the above components are dissolved in solvent such as toluene, hexane, methylene chloride or the like using a mixing machine such as a mixer with anti-explosion treatment, or the like. The obtained solution is spread on a paper, a film, or the like in which a removable treatment is carried out, dried by a drying machine to remove solvent, and then transferred to a desirable backing in a covered state by compression. A removable cover is stuck on the spread coat on the backing to obtain a plaster of the invention. As such a backing, specifically, examples include cloth, non-woven cloth, polyurethane, polyester, polyvinyl acetate, polyvinylidene chloride, polyethylene, polyethylene terephthalate, aluminum sheet, or a composite material thereof, and the like. In addition, as to the removable cover, examples include those consisting of polyethylene, polypropylene, polyvinyl chloride, polyester, polyvinylidene chloride and paper treated with silicone, and the like.

Next, an ointment is explained. An ointment base used in the ointment of the invention is not particularly limited and may be selected among those conventionally used. As components contained in such an ointment base, examples include higher fatty acids or esters thereof (adipic acid, myristic acid, palmitic acid, stearic acid, oleic acid, adipate, myristate, palmitate, diethyl sebacate, hexyl laurate, cetyl isooctate, etc.), wax (whale wax, beeswax, ceresin, etc.), surfactants (polyoxyethylene alkyl ether phosphate, etc.), higher alcohols (cetanol, stearyl alcohol, cetostearyl alcohol, etc.), silicone oils (dimethylpolysiloxane, methylphenylpolysiloxane, glycol methylpolysiloxane, silicone glycol polymer, etc.), hydrocarbons (hydrophilic petrolatum, white petrolatum, purified lanolin, liquid paraffin, etc.), water, absorption enhancers (propylene carbonate, diisopropyl adipate, crotamiton, Azone(R), etc.), moisturizing agents (glycerin, propylene glycol, butylene glycol, sorbitol, etc), irritation inhibitors, other additives (salicylic acid, methyl salicylate, ethyleneglycol salicylate, 1-menthol, camphor, peppermint oil, etc.) and the like, and by blending the above described essential components with an ointment base prepared by mixing various components selected among these, the ointments of the invention can be obtained.

Next, one favorable production example of ointments (formulation example) is shown. Namely, first, the above NSAID 0.1-10 parts by weight, and the above UV blocker 0.01-20 parts by weight are mixed with the higher fatty acid ester 5-15 parts by weight and the surfactant 1-10 parts by weight at room temperature or under heating, added with the wax 4-10 parts by weight and the hydrocarbon 50-90 parts by weight, and heated to keep 50-100°C. After all the components become a clear solution, it is homogeneously mixed by a homo-mixer. Then, by letting the obtained mixture cool to room temperature under stirring, an ointment of the invention can be prepared.

Next, a gel is explained. A gel base used in the ointment of the invention is not particularly limited and may be selected among those conventionally used. As components contained in such a gel base, examples include lower alcohols (ethanol, isopropanol, etc.), water, gelatinizing agents (carboxyvinyl copolymer, hydroxyethyl cellulose, hydroxypropyl cellulose, methyl cellulose, ethyl cellulose, carboxymethyl cellulose, propylene glycol alginate, etc.), neutralizing agents (triethanolamine, diisopropanolamine, sodium hydroxide, etc.), surfactants (sorbitan sesquioleate, sorbitan trioleate, sorbitan monooleate, sorbitan monostearate, sorbitan monolaurate, polyethylene glycol monostearate, polyoxyethylene nonylphenyl ether, polyoxyethylene cetyl ether, polyoxyethylene lauryl ether, etc.) and absorption enhancers (propylene carbonate, diethyl sebacate, diisopropyl adipate, crotamiton, Azone(R), propylene glycol, etc), solubilizers (lower alcohols such as ethanol and isopropanol, higher alcohols such as cetyl alcohol, stearyl alcohol, batyl alcohol, behenyl alcohol, oleyl alcohol, hexadecyl alcohol, and octyldodecanol, fatty acid esters such as isopropyl myristate, octyldodecyl myristate, cetyl myristate, myristyl myristate, diethyl sebacate, diisopropyl sebacate, diisopropyl adipate, oleyl oleate, hexyl laurate, cetyl isooctanoate, medium chain fatty acid triglyceride and propylene glycol fatty acid ester, N-methyl-2-pyrrolidone, triacetin, benzyl alcohol, lanolin alcohol, 1-menthylglyceryl ether, etc.), glycols (glycerin, propylene glycol, polyethylene glycol, polypropylene glycol, sorbitol, 1,3-butylene glycol, dipropylene glycol, etc.), irritation inhibitors, other additives (salicylic acid, methyl salicylate, ethyleneglycol salicylate, 1-menthol, camphor, peppermint oil, etc.) and the like, and by blending the above described essential components with a gel base prepared by mixing various components selected among these, the gels of the invention can be prepared.

Next, one favorable production example of gels (formulation example) is shown. Namely, first, the gelatinizing agent 0.5-5 parts by weight was added to water not more than 55 parts by weight, and swelled to obtain swelled product A. In the meantime, the above NSAID 0.1-10 parts by weight, and the above UV blocker 0.01-20 parts by weight are dissolved or suspended in the solubilizer 0.1-10 parts by weight, and further, this is dissolved in a mixture of the glycol not more than 40 parts by weight and the lower alcohol not more than 60 parts by weight to obtain solution B. Then, the solution B is added to the swelled product A and followed by addition of the neutralizing agent so as to adjust the pH value to 4-7 to obtain a gel of the invention.

Next, a cream is explained. A cream base used in the cream of the invention is not particularly limited and may be selected among those conventionally used. As components contained in such a cream base, examples include higher fatty acid esters (adipate, myristate, palmitate, diethyl sebacate, hexyl laurate, cetyl isooctanoate, etc.), lower alcohols (ethanol, isopropanol, etc.), hydrocarbons (liquid paraffin, squalane, etc.), polyhydric alcohols (propylene glycol, butylene glycol, etc.), higher alcohols (2-hexyldecanol, cetanol, 2-octyldodecanol, etc.), emulsifiers (polyoxyethylene alkyl ethers, fatty acid esters, polyethylene glycol fatty acid ester, etc.), preservatives (*p*-hydroxybenzoic acid ester, etc.), absorption enhancers (propylene carbonate, diethyl sebacate, diisopropyl adipate, crotamiton, Azone(R), propylene glycol, etc.), irritation inhibitors, other additives (salicylic acid, methyl salicylate, ethyleneglycol salicylate, 1-menthol, camphor, peppermint oil, etc.) and the like, and by blending the above described essential components with a cream base prepared by mixing various components selected among these, the creams of the invention can be prepared. In addition, in order to prepare a gelatinized cream having intermediate properties between a gel and a cream, to the above cream is added a gelatinizing agent (carboxyvinyl copolymer, hydroxyethyl cellulose, hydroxypropyl cellulose, methyl cellulose, ethyl cellulose, carboxymethyl cellulose, propylene glycol alginate, etc.), a neutralizing agent (triethanolamine, diisopropanolamine, sodium hydroxide, etc.) to obtain a gelatinized cream of the invention by adjusting the pH value to 4-8 (preferably 5-6.5).

Next, one favorable production example (formulation example) of gelatinized creams is shown. Namely, first, the above NSAID 0.1-10 parts by weight, and the above UV blocker 0.01-20 parts by weight are dissolved in a mixture of the higher fatty acid ester not more than 25 parts by weight and the lower alcohol not more than 40 parts by weight and further added with the preservative not more than 0.5 parts by weight and the emulsifier not more than 5 parts by weight to obtain mixture A. In the meantime, the gelatinizing agent was added to water so that the concentration becomes 0.5-5 parts by weight, and swelled to obtain swelled product B. Then, the swelled product B is added to the mixture A, homogeneously emulsified by a homo-mixer, and followed by addition of the neutralizing agent to the obtained emulsified product so as to adjust the pH value to 4-8 to obtain a gelatinized cream of the invention.

Next, a liniment is explained. A liniment base used in the liniment of the invention is not particularly limited and may be selected among those conventionally used. As components contained in such a liniment base, examples include alcohols (monohydric alcohols such as ethanol, propanol and isopropanol, polyhydric alcohols such as polyethylene glycol, propylene glycol and butylene glycol, etc.) 10-70 parts by weight, fatty acid esters (various esters of adipic acid, sebacic acid and myristic acid, etc.) not more than 60 parts by weight, and surfactants (polyoxyethylene alkyl ether, polyoxyethylene hardened caster oil, etc.) not more than 10 parts by weight, and by blending the above NSAID 0.1-10 parts by weight, and the above UV blocker 0.01-20 parts by weight with such a liniment base, a liniment of the invention can be obtained. Further, in the liniment of the invention, if necessary, a neutralizing agent for adjustment of the pH value, a thickener such as methyl cellulose, an irritation inhibitor, and other additives (salicylic acid, methyl salicylate, ethyleneglycol salicylate, 1-menthol, camphor, peppermint oil, red pepper extract, vanillic amide nonylate, crotamiton, Azone(R), propylene carbonate, diisopropyl adipate, etc.) and the like may be blended.

The above formulation example and production method are simply examples, and therefore, the liniment of the invention can be prepared by a known production method for a liniment. In addition, also in a blend composition, an efficacious component in a known liniment is replaced by ketoprofen and the like, whereby a liniment of the invention can easily be obtained by blending the UV blocker.

As described above, preferable embodiments of bases and formulation examples according to each dosage form of the transdermal preparations for external use of the invention are explained, although, the dosage forms and formulation examples are not limited to these, and the blending sequence of each component is not particularly limited. For example, in a formulation of conventionally known eyedrop or a formulation of conventionally known aerosol, an efficacious component is replaced by the above NSAID 0.1-10 parts by weight, and further, the above UV blocker 0.01-20 parts by weight is blended to obtain an eyedrop or an aerosol of the invention.

Additionally, in the transdermal preparation for external use of the invention, an antioxidant may further be blended in addition to the above formulation. As such an antioxidant preference is given to phenol derivatives such as *tert*-butyl hyroxyanisole, di-*tert*-butylhydroxytoluene, thymol and propyl gallate, tocopherol and its ester derivatives, ascorbic acid and its ester derivatives, etc. Such antioxidants may be used alone, or two or more members may be used in combination. Although the blend amount is not particularly limited, it is preferably 0-10 weight %, and more preferably 0-5 weight %, based on the total amount of the preparation.

### [Example]

In the following, the invention is explained in more detail by examples. Here, in the following examples, "%" means "weight %" unless otherwise specified.

### Example 1 (Murine auricular photo-irradiation test)

The following experiments were carried out by referring to the method of Gerberick et al. (Food Chem. Toxicol. , 27, 813-819 (1989)) and modifying a part of it. Namely, as a test animal, Balb/c mice (female, 9-11 weeks old) were used. Each test substance {4-*tert*-butyl-4'-methoxydibenzoylmethane (BM-DBM), 2-ethylhexyl dimethoxybenzylidenedioxoimidazolidinepropionate (DB-DIH), terephthalylidene-3,3'-dicamphor-10,10'-disulfonic acid (TC-DCS) and n-hexyl 2-(4-dimethylamino-2-hydroxybenzoyl)benzoate (DHB-BH)} which were dissolved in ethanol and 2% ketoprofen (KP) were applied on the auricle, and followed by UVA irradiation at 40J/cm².
The ear thickness was measured 24 hours after the UVA irradiation, and an increase from the ear thickness prior to the start of the test was calculated. A suppression effect of each test substance for the skin symptom due to a non-immunological mechanism resulting from ketoprofen upon photo-irradiation was evaluated by an indicator which shows the degree of inhibition of the increase in the ear thickness due to KP, that is, the ear edema suppression ratio (%) toward the KP group. The obtained results are shown in Tables 1 and 2.

**[Table 1]**

| Test substance (conc.: 0.5%) | Ear edema suppression ratio (%) |
|---|---|
| BM-DBM | 98 |
| DB-DIH | 34 |
| TP-DCS | 65 |
| DHB-BH | 84 |

**[Table 2]**

| Test substance (conc.: %) | Ear edema suppression ratio (%) |
|---|---|
| BM-DBM (0.1) | 41 |
| BM-DBM (0.3) | 81 |
| BM-DBM (0.5) | 98 |

As is evident from the results shown in Tables 1 and 2, it was confirmed that the skin symptom due to the non-immunological mechanism upon photo-irradiation was remarkably reduced when the UV blocker migrating into the skin was blended in a transdermal preparation for external use which contains ketoprofen as an efficacious component.

### Example 2 (Murine local lymph node test 1)

Detection of a skin symptom due to an immunological mechanism resulting from KP upon photo-irradiation was carried out by a modified method of Murine Local Lymph Node Assay (LLNA) proposed by The Interagency Coordinating Committee on the Validation of Alternative Methods (ICCVAM). Namely, as a test animal, Balb/c mice (female, 8-12 weeks old) were used. The test substance {4-*tert*-butyl-4' -methoxydibenzoylmethane (BM-DBM)} and 2% ketoprofen (KP) which were dissolved in acetone - olive oil (4:1, v/v) were applied on the back of the pinna, and followed by UVA irradiation at 20J/cm². This treatment inducing sensitization was carried out for 3 days consecutively. After 5 days from the start of sensitization, a ³H-methylthymidine (³H-TdR)/PBS solution of 20µCi per mouse was injected in the tail vein, and after 5 hours, the auricular lymph node was removed. The removed lymph node was made into a single lymph cell suspension by a cell strainer, then washed two times with PBS, and let it stand in 5% trichloroacetic acid (TCA) solution at 4°C overnight to precipitate DNA. The precipitate was suspended in 1ml of 5% TCA solution and a radiation activity was measured by a liquid scintillation counter. A suppressive effect by the test substance toward the above skin symptom resulting from KP was evaluated by an indicator which shows the degree of suppression of the increase in ³H-TdR uptake due to KP, that is, the ³H-TdR uptake suppression ratio toward the KP group. The obtained results are shown in Table 3.

**[Table 3]**

| BM-DBM concentration (%) | ³H-TdR uptake suppression ratio(%) |
|---|---|
| 0.06 | 30 |
| 0.13 | 51 |
| 0.25 | 24 |
| 0.50 | 94 |
| 1.00 | 99 |

As is evident from the results shown in Table 3, it was confirmed that the skin symptom due to the immunological mechanism upon photo-irradiation was remarkably reduced by blending the UV blocker migrating into the skin in a transdermal preparation for external use which contains ketoprofen as an efficacious component.

### Example 3: (Photo-sensitization test using guinea pigs)

An effect exerted by the UV blocker to skin symptoms due to an immunological mechanism resulting from KP upon photo-irradiation was further examined by a modified method of the Adjuvant and Strip method of Sato et al. (Nishinihon Hifuka, 42, 831-837 (1980)) using guinea pigs. That is, the cervical back of a white female Hartley strain guinea pig (eight guinea pigs per group) was depilated, and the adjuvant was administered to four corners of 2 x 2cm, and then, 2% ketoprofen (KP) solution, or 2% KP and 2% test substance {4-*tert*-butyl-4'-methoxydibenzoylmethane (BM-DBM)} solution, which were dissolved in ethanol, were applied on the above parts of 2 x 2cm under an open condition, and after 1 hour, UVA (10 J/cm²) was irradiated. This treatment of sensitization induction was carried out successively for 5 days. After 3 weeks from the start of the sensitization, the lumber back was depilated, and the area of 2 x 2cm were applied by the same solutions under the open condition, and then, after 1 hour, photo-induction was carried out by UVA irradiation (10 J/cm²). Skin reactions after 24 hours and 48 hours from the irradiation were evaluated according to the above standard of Sato et al. The suppression effect by BM-DBM to the skin symptoms due to the immunological mechanism resulting from KP upon photo-irradiation was evaluated by the erythema/edema suppression ratio (%) toward the KP group. The obtained results are shown in Table 4.

**[Table 4]**

| Test substance | Erythema/Edema suppression ratio (%) | |
|---|---|---|
| | After 24 hrs | After 48 hrs |
| BM-DBM | 69 | 74 |

As is evident from the results shown in Table 4, it was confirmed that the skin symptoms were remarkably reduced by blending the UV blocker migrating into the skin in a transdermal preparation for external use which contains ketoprofen as an efficacious component.

### Example 4 (Murine auricular lymph node test 2)

Detection of a skin symptom due to an immunological mechanism resulting from KP upon photo-irradiation was carried out by a modified method of the above LLNA. Namely, as a test animal, Balb/c mice (female, 8-12 weeks old) were used. A test substance of 2% concentration and KP of 2% concentration which were dissolved in acetone - olive oil (4:1, v/v) or acetone - olive oil - physiological saline (3:3:1, v/v) were applied on the back of the pinna, and followed by UVA irradiation at 20J/cm². This treatment inducing sensitization was carried out for three days consecutively. After 5 days from the start of the sensitization, the murine auricular lymph node was removed, and pooled in PBS. Then, PBS attached to the removed lymph node was sufficiently wiped up, and weighed. A suppressive effect by the test substance on the skin symptom due to the immunological mechanism was evaluated by an indicator which shows the degree of suppression in the weight increase of the auricular lymph node due to the above-described symptom, that is, the suppression ratio (%) in the weight increase of the auricular lymph node toward the KP group. The obtained results are shown in the following.

**[Table 5]**

| Name of test substance | Suppression ratio (%) |
|---|---|
| 4-*tert*-Butyl-4'-methoxydibenzoylmethane | 88 |
| 2-(2'-Hydroxy-5'-methoxyphenyl)-benzotriazole | 4 |
| Terephthalylidene-3,3'-dicamphor-10,10'-disulfonic acid | 63 |
| 2,2',4,4'-Tetrahydroxybenzophenone | -32 |
| Oxybenzone | 56 |
| Ethyl 4-aminobenzoate | 43 |
| 4-Aminobenzoic acid | -39 |
| 2-Ethylhexyl 4-methoxycinnamate | 20 |
| Phenyl salicylate | 0 |
| Ethylene glycol monosalicylate | -68 |

As is evident from the results shown in Table 5, it was confirmed that the above skin symptom was remarkably reduced by blending the UV blocker migrating into the skin in a transdermal preparation for external use which contains ketoprofen as an efficacious component.

In the following, the formulation examples of the transdermal preparation for external use of the invention are described.

### Formulation example 1 (Plaster)

16 parts by weight of a styrene-isoprene-styrene block copolymer (SIS5200P: manufactured by JSR Corporation), 10 parts by weight of polyisobutylene (L-100: manufactured by Exxon Mobil), 19 parts by weight of a petroleum resin (Arkon P-70: manufactured by Arakawa Chemical Industry Co., Ltd.), 45 parts by weight of liquid paraffin (Crystol J-352: manufactured by Esso Petroleum Ltd.), and 1.99 parts by weight of synthetic aluminum silicate were stirred under an atmosphere of nitrogen gas while heating to give a melt product (Step A). The stirring temperature was 110-200°C, and the stirring time was 30-120 minutes. Subsequently, 3 parts by weight of crotamiton, 0.01 parts by weight of 4-*tert*-butyl-4'-methoxydibenzoylmethane, 2 parts by weight of ketoprofen, and 3 parts by weight of 1-menthol were added to the above-described melt product so that the stirring temperature was in the range of 110-200°C, and mixed for 5-30 minutes to give a homogeneous melt product, which was a base for a plaster (Step B). Said base was spread on a silicone-treated polyester film at a weight of 1 g per 70cm², then covered with a polyester woven cloth, transferred by compression, and cut to a desired size to give a plaster of the invention.

### Formulation examples 2-13 (Plaster).

Plasters of the invention were obtained in the same manner as the formulation example 1 using the components and the blend amounts shown in Table 6.

**[Table 6]**

| | Formulation example 2 | Formulation example 3 | Formulation example 4 | Formulation example 5 | Formulation example 6 | Formulation example 7 | Formulation example 8 | Formulation example 9 | Formulation example 10 | Formulation example 11 *** | Formulation example 12 *** | Formulation example 13 |
|---|---|---|---|---|---|---|---|---|---|---|---|---|
| Ketoprofen* | 2.00 | 2.00 | 2.00 | 2.00 | 2.00 | 2.00 | 2.00 | 2.00 | 2.00 | 2.00 | 2.00 | |
| Diclofenac* | | | | | | | | | | | | 2.00 |
| methoxydibenzoylmethane* | | 5.00 | 20.00 | 3.00 | 3.00 | | 3.00 | | | | | 5.00 |
| n-Hexyl 2-(4-diethylamino-(2-hydroxybonzoyl)benzoate* | 3.00 | | | | | 3.00 | | | | | | |
| 2-(2-Hydroxy-5-methoxyphenyl)-benzotriazole* | | | | | | | | 3.00 | | | | |
| Oxybenzone | | | | | | | | | | | | |
| Ethyl 4-aminobenzoate* | | | | | | | | | 3.00 | 3.00 | | |
| 2-Ethylhexyl 4-methoxyoinnamate* | | | | | | | | | | | 5.00 | |
| Styrene-isoprene-styrene block copolymer** | 15.00 | 17.00 | 15.00 | 15.00 | 15.00 | 15.00 | 15.00 | 15.00 | 15.00 | 15.00 | 17.00 | 26.00 |
| Polyisobutylene** ** | 9.00 | 9.00 | 8.00 | 10.00 | 10.00 | 8.00 | 8.00 | 10.00 | 8.00 | 8.00 | 9.00 | 9.00 |
| Petroleum resin | | | | | | | | | | | | 20.00 |
| Liquid paraffin** | 50.00 | 50.00 | 40.00 | 50.00 | 45.00 | 50.00 | 50.00 | 45.00 | 50.00 | 50.00 | 50.00 | 35.00 |
| Synthetic aluminum silicate** | | | | | | 1.49 | | | | | | 3.00 |
| Maleic acid hydrogenated resin esther ** | 14.00 | 11.00 | 10.00 | 13.00 | | 15.00 | | | | | 11.00 | |
| Sodium stearate ** | 2.00 | | | | | | | | | | | |
| Zinc stearate ** | | 1.00 | | | 2.00 | | 4.00 | | 4.00 | 4.00 | 1.00 | |
| Magnesium stearate ** | | | 1.00 | | | | | 2.00 | | | | |
| Calcium stearate ** | | | | 1.99 | | | | | | | | |
| Hydrogenated resin glycerin ester ** | | | | | 13.00 | | 10.00 | 13.00 | 10.00 | 10.00 | | |
| Crotamiton * | | | | 2.00 | 2.00 | | | 2.00 | | | | |
| 1-Menthol * | 3.00 | 3.00 | 3.00 | 3.00 | 3.00 | 3.00 | 3.00 | 3.00 | 3.00 | 3.00 | 3.00 | |
| Diethyl sebacate * | 2.00 | | | | | | | | | | | |
| Propylene glypol * | | 2.00 | | | | | | | | | 2.00 | |
| Diisopropyl adipate * | | | 1.00 | | | 1.00 | | | | | | |
| Propyl gallate * | | | | 0.01 | | 0.01 | 2.00 | | 2.00 | 2.00 | | |
| Di-tert-buthylhydroxytoluene * | | | | | 5.00 | 1.50 | 3.00 | 5.00 | 3.00 | 3.00 | | |
| Dipropylene glycol * | | | | | | | | | 1.50 | 1.50 | | 1.00 |
| Total amount (wt%) | 100.00 | 100.00 | 100.00 | 100.00 | 100.00 | 100.00 | 100.00 | 100.00 | 100.00 | 1000.00 | 100.00 | 100.00 |

| | | | | | | | | | | | | |
|---|---|---|---|---|---|---|---|---|---|---|---|---|
| * Added in Step B ** Mixed in Step A *** Reference example | | | | | | | | | | | | |

### Formulation example 14 (Plaster)

2-Ethylhexyl acrylate 50 parts by weight, methoxyethyl acrylate 25 parts by weight, vinyl acetate 14.7 parts by weight, azobisisobutyronitrile 0.3 parts by weight, synthetic aluminum silicate 3 parts by weight, and ethyl acetate 100 parts by weight were placed in a reaction vessel; polymerization was initiated by warming the mixture to 65°C under a nitrogen atmosphere, reacted for 10 hours, and further aged at 80°C for 2 hours to obtain a copolymer solution. The obtained copolymer solution was added and mixed with 4-*tert*-butyl-9'-methoxydibenzoylmethane 5 parts by weight and ketoprofen 2 parts by weight to obtain a plaster base as a homogeneous mix solution. The obtained base was spread on a silicone-treated polyester film at a weight of 1 g per 70cm², covered with a polyester woven cloth after ethyl acetate was evaporated by a hot blast, transferred by compression, and cut to a desired size to give a plaster of the invention.

### Formulation example 15 (Plaster)

2-Ethylhexyl acrylate 45 parts by weight, methoxyethyl acrylate 25 parts by weight, vinyl pyrrolidone 12 parts by weight, benzoyl peroxide 1 parts by weight, synthetic aluminum silicate 3 parts by weight, 4-*tert*-butyl-4'-methoxydibenzoylmethane 5 parts by weight, di-*tert*-butylhydroxytoluene 5 parts by weight, and ethyl acetate 100 parts by weight were placed in a reaction vessel; polymerization was initiated by warming the mixture to 65°C under a nitrogen atmosphere, reacted for 10 hours, and further aged at 80°C for 2 hours to obtain a copolymer solution. The obtained copolymer solution was added and mixed with ketoprofen 4 parts by weight to obtain a plaster base as a homogeneous mix solution. The obtained base was spread on a silicone-treated polyester film at a weight of 1 g per 70cm², covered with a polyester woven cloth after ethyl acetate was evaporated by a hot blast, transferred by compression, and cut to a desired size to give a plaster of the invention.

### Formulation example 16 (Poultice)

Ketoprofen 0.3 parts by weight, 4-*tert*-butyl-4' -methoxydibenzoylmethane 1 part by weight, and crotamiton 0.5 parts by weight were mixed and dissolved to obtain homogeneous mixture A. In the meantime, sodium polyacrylate 6 parts by weight, polyacrylic acid 3 parts by weight, gelatin 2 parts by weight, and polyvinyl alcohol 1 part by weight were mixed, dispersed and dissolved with glycerin 20 parts by weight, sorbitol 5 parts by weight, and water 59.6 parts by weight, and further added with synthetic aluminum silicate 0.5 parts by weight, dihydroxyaluminum aminoacetate 0.1 parts by weight, magnesium metasilicate aluminate 0.3 parts by weight, and tartaric acid 0.2 parts by weight to obtain homogeneous kneaded product B. Then, the mixture A was added to the kneaded product B to obtain a homogeneous kneaded product. After the obtained kneaded product was expansively coated on a polyester non-woven cloth, a polypropylene film was stuck on it to obtain a poultice of the invention.

### Formulation examples 17-19 (Poultice)

Poultices of the invention were obtained in the same manner as the formulation example 16 using the components and the blend amounts shown in Table 7.

**[Table 7]**

| (wt%) | | | |
|---|---|---|---|
| | Formulation example 17 | Formulation example 18 | Formulation example 19 |
| Diclofenac | 0.5 | 5.0 | |
| Ibuprofen | | | 10.0 |
| 4-*tert*-Butyl-4'-methoxydibenzoylmethane | 3.0 | | |
| 2-(2H-benzotriazol-2-yl)-4-methyl-6-[2-methyl-3-[1,3,3,3-tetramethyl-1-(trimethylsilyl]oxy]-disiloxanyl]-propyl]phenol | | 10.0 | 15.0 |
| Crotamiton | 3.0 | 5.0 | 8.0 |
| 1-Menthol | 0.5 | 0.5 | 0.5 |
| Sodium polyacrylate | 6.0 | 6.0 | 6.0 |
| Polyacrylic acid | 3.0 | 3.0 | 3.0 |
| Gelatin | 2.0 | 2.0 | 2.0 |
| Polyvinyl alcohol | 1.0 | 1.0 | 1.0 |
| Glycerin | 20.0 | 20.0 | 20.0 |
| Sorbitol | 5.0. | 5.0 | 5.0 |
| Synthetic aluminum silicate | 0.5 | 0.5 | 0.5 |
| Dihydroxyaluminum aminoacetate | 0.1 | 0.1 | 0.1 |
| Magnesium metasilicate aluminate | 0.3 | 0.3 | 0.3 |
| Tartaric acid | 0.2 | 0.2 | 0.2 |
| Water | 54.9 | 41.4 | 28.4 |

### Formulation example 20 (Ointment)

Ketoprofen 2 parts by weight and 4-*tert*-butyl-4'-methoxydibenzoylmethane 0.4 parts by weight were mixed with diethyl sebacate 4.7 parts by weight and glycerol monooleate 4.4 parts by weight at room temperature or under heating, added with beeswax 6.8 parts by weight, liquid paraffin 8.1 parts by weight, and white petrolatum 73.6 parts by weight, and heated to keep 50-100°C. After all the components became a clear solution, the solution was homogeneously mixed by a homo-mixer. Then, the obtained mixture was let cool to room temperature under stirring to obtain an ointment of the invention.

### Formulation examples 21-25 (Ointment)

Ointments of the invention were obtained in the same manner as the formulation example 20 using the components and the blend amounts shown in Table 8.

**[Table 8]**

| | Formulation example 21 | Formulation example 22 | Formulation example 23 | Formulation example 24 | Formulation example 25 |
|---|---|---|---|---|---|
| Ketoprofen | 2.0 | | | | |
| Suprofen | | 3.0 | 3.0 | | |
| Piroxicam | | | | 1.0 | 1.0 |
| 4-*tert*-Butyl-4'-methoxydibenzoylmethane | 2.1 | | | | |
| Terephthalylidene -3,3' -dicamphor-10,10'-disulfonic acid | | 0.2 | 5.3 | | |
| n-Hexyl 2-(4-diethylamino-2-hydroxybenzoyl) -benzoate | | | | 1.4 | 17.6 |
| Diethyl sebacate | 4.7 | 4.7 | 4.7 | 4.7 | 4.7 |
| Glycerol monooleate | 4.4 | 4.4 | 4.4 | 4.4 | 4.4 |
| Beeswax | 6.8 | 6.8 | 6.8 | 6.8 | 6.8 |
| Liquid paraffin | 8.1 | 8.1 | 8.1 | 8.1 | 8.1 |
| White petrolatum | 71.9 | 72.8 | 67.7 | 73.6 | 57.4 |
| Total amount (wt%) | 100.0 | 100.0 | 100.0 | 100.0 | 100.0 |

### Formulation example 26 (Gel)

Carboxyvinyl polymer 1.8 parts by weight was added to a purified water 30.2 parts by weight and swelled to obtain swelled product A. In the meantime, tiaprofenic acid 3 parts by weight, isostearyl 4-hydroxy-3-methoxycinnamate 2.5 parts by weight and dibutylhydroxytoluene 0.5 parts by weight were dissolved in a mixture of propylene glycol 13.7 parts by weight and anhydrous ethanol 42.5 parts by weight to obtain solution B. Then, after the solution B was added to the swelling product A, triethanolamine 0.9 parts by weight was added to adjust the pH value, whereby a gel of the invention was obtained

### Formulation examples 27-33 (Gel)

Gels of the invention were obtained in the same manner as the formulation example 26 using the components and the blend amounts shown in Table 9.

**[Table 9]**

| | Formulation example 27 | Formulation example 28 | Formulation example 29 | Formulation example 30 | Formulation example 31 | Formulation example 32 | Formulation 33 example |
|---|---|---|---|---|---|---|---|
| Tiaprofenic acid | 3.00 | | | | | | |
| Tolmetin | | 1.00 | 1.00 | | | | |
| Naproxen | | | | 5.00 | 5.00 | | |
| Diflunisal | | | | | | 2.00 | 2.00 |
| Isostearyl 4-hydroxy-3-methoxycinnamate | 8.40 | | | | | | |
| 4-Hydroxy-3-methoxycinnamic acid | | 0.04 | 3.90 | | | | |
| 2-Ethylhexyl dimethoxybenzylidene-dioxoimidazolidinepropionate | | | | 4.20 | 12.20 | | |
| 1-(3,4-Dimethoxyphenyl)-4,4-dimethyl-1,1-pentanedione | | | | | | 1.60 | 7.30 |
| Anhydrous ethanol | 42.50 | 42.50 | 42.50 | 42.50 | 42.50 | 42.50 | 42.50 |
| Propylene glycol | 13.70 | 13.70 | 13.70 | 13.70 | 13.70 | 13.70 | 13.70 |
| Diisopropyl adipate | 4.90 | 4.90 | 4.90 | 4.90 | 4.90 | 4.90 | 4.90 |
| Dibutylhydroxytoluene | 0.50 | 0.50 | 0.50 | 0.50 | 0.50 | 0.50 | 0.50 |
| Carboxyvinyl polymer | 1.80 | 1.80 | 1.80 | 1.80 | 1.80 | 1.80 | 1.80 |
| Triethanolamine | 0.90 | 0.90 | 0.90 | 0.90 | 0.90 | 0.90 | 0.90 |
| Purified water | 24.30 | 34.65 | 30.80 | 26.50 | 18.50 | 32.10 | 26.40 |
| Total amount (wt%) | 100.00 | 100.00 | 100.00 | 100.00 | 100.00 | 100.00 | 100.00 |

### Formulation examples 34-39 (Cream)

Creams of the invention were obtained by mixing the components shown in Table 10 at the blend amount indicated in the same table.

**[Table 10]**

| | Formulation example 34 | Formulation example 35 | Formulation example 36 | Formulation example 37 | Formulation example 38 | Formulation example 39 |
|---|---|---|---|---|---|---|
| Carprofen | 3.00 | 3.00 | | | | |
| Benoxaprofen | | | 1.00 | 1.00 | | |
| Benzydamine | | | | | 5.00 | 5.00 |
| Terephthalylidene-3,3'dicamphor-10,10' disulfonic acid | 2.50 | 8.40 | | | | |
| n-Hexy1 2-(4-diethylamino-2-hydroxybenzoyl)benzoate | | | 0.04 | 3.90 | | |
| Isostearyl 4-hydroxy-3-methoxycinnamate | | | | | 4.20 | 12.20 |
| Diethyl sebacate | 3.50 | 3.50 | 3.50 | 3.50 | 3.50 | 3.50 |
| Cetanol | 9.60 | 9.60 | 9.60 | 9.60 | 9.60 | 9.60 |
| Liquid paraffin | 6.80 | 6.80 | 6.80 | 6.80 | 6.80 | 6.80 |
| White petrolatum | 12.40 | 12.40 | 12.40 | 12.40 | 12.40 | 12.40 |
| Dibutylhydroxytoluene | 0.30 | 0.30 | 0.30 | 0.30 | 0.30 | 0.30 |
| Polyoxyethylene stearyl ether | 4.30 | 4.30 | 4.30 | 4.30 | 4.30 | 4.30 |
| Methyl p-oxybenzoate | 0.20 | 0.20 | 0.20 | 0.20 | 0.20 | 0.20 |
| Purified water | 57.40 | 51.50 | 61.86 | 58.00 | 53.70 | 45.70 |
| Total amount (wt%) | 100.00 | 100.00 | 100.00 | 100.00 | 100.00 | 100.00 |

### Formulation examples 40-47 (Liniment)

Liniments of the invention were obtained by mixing the components shown in Table 11 at the blend amount indicated in the same table.

**[Table 11]**

| | Formulation example 40 | Formulation example 41 | Formulation example 42 | Formulation example 43 | Formulation example 44 | Formulation example 45 | Formulation example 46 | Formulation example 47 |
|---|---|---|---|---|---|---|---|---|
| Diclofenac | 5.0 | 5.0 | | | | | | |
| Naproxen | | | 2.0 | 2.0 | | | | |
| Ketoprofen | | | | | 3.0 | 3.0 | | |
| Diflunsal | | | | | | | 1.0 | 1.0 |
| n-Hexyl 2-(4-diethylamino-2-hydroxybenzoyl)benzoate | 0.7 | 3.2 | | | | | | |
| 4-*tert*-Butyl-4'-methoxydibenzoylmethane | | | 4.8 | 13.9 | | | | |
| 2-Ethylhexyl dimethoxybenzylidenedioxoimidazolidinepropionate | | | | | 2.7 | 9.4 | | |
| Isostearyl 4-hydroxy-3-methoxycinnamate | | | | | | | 1.7 | 7.3 |
| Anhydrous ethanol | 48.8 | 48.8 | 48.8 | 48.8 | 48.8 | 48.8 | 48.8 | 48.8 |
| Propylene glycol | 7.8 | 7.8 | 7.8 | 7.8 | 7.8 | 7.8 | 7.8 | 7.8 |
| Diethyl sebacate | 3.1 | 3.1 | 3.1 | 3.1 | 3.1 | 3.1 | 3.1 | 3.1 |
| Polyoxyethylene hardened castor oil | 2.2 | 2.2 | 2.2 | 2.2 | 2.2 | 2.2 | 2.2 | 2.2 |
| Dibutylhydroxytoluene | 0.2 | 0.2 | 0.2 | 0.2 | 0.2 | 0.2 | 0.2 | 0.2 |
| Purified water Total amount | 32.2 | 29.7 | 31.1 | 22.0 | 32.2 | 25.5 | 35.2 | 29.6 |
| (wt%) | 100.0 | 100.0 | 100.0 | 100.0 | 100.0 | 100.0 | 100.0 | 100.0 |

### [Industrial Applicability]

As described above, in a transdermal preparation for external use which contains a photosensitive NSAID, it becomes possible according to the invention to exhibit anti-inflammatory/analgesic effects while surely inhibiting skin symptoms due to non-immunological and immunological mechanisms resulting from the above described component upon photo-irradiation, and application as a medicine which is extremely high in stability can be expected.

## Claims

1. A transdermal preparation for external use which contains a photosensitive NSAID and a UV blocker migrating into the skin, wherein the UV blocker is selected from the group consisting of 4-*tert*-butyl-4'-methoxydibenzoylmethane, n-hexyl 2-(4-diethyl-amino-2-hydroxybenzoyl)benzoate, 4-hydroxy-3-methoxycinnamic acid, a branched alkyl ester of 4-hydroxy-3-methoxycinnamic acid, terephthalylidene-3,3'-dicamphor-10,10'-disulfonic acid, 2-(2H-benzotriazol-2-yl)-4-methyl-6-[2-methyl-3-[1,3,3,3-tetramethyl-1-[(trimethylsilyl)oxy]disiloxanyl]propyl]phenol, 2-ethylhexyl dimethoxybenzylidenedioxoimidazolidinepropionate, 1-(3,4-dimethoxyphenyl)-4,4-dimethyl-1,3-pentanedione, 2-(2'-hydroxy-5'-methoxyphenyl)-benzotriazole, and oxybenzone.

2. The transdermal preparation for external use according to claim 1, wherein the NSAID is selected from the group consisting of ketoprofen, tiaprofenic acid, suprofen, tolmetin, carprofen, benoxaprofen, piroxicam, benzydamine, naproxen, diclofenac, ibuprofen, diflunisal, azapropazone, and pharmaceutically acceptable salts thereof.

## Patentansprüche

1. Transdermale Zubereitung zur äußerlichen Anwendung, die ein lichtempfindliches NSAID und einen in die Haut migrierenden UV-Blocker enthält, wobei der UV-Blocker ausgewählt ist aus der Gruppe, bestehend aus 4-*tert*-Butyl-4'-methoxydibenzoylmethan, n-Hexyl-2-(4-diethylamino-2-hydroxybenzoyl)benzoat, 4-Hydroxy-3-methoxyzimtsäure, einem verzweigten Alkylester von 4-Hydroxy-3-methoxyzimtsäure, Terephthalyliden-3,3'-dicampher-10,10'-disulfonsäure, 2-(2H-Benzotriazol-2-yl)-4-methyl-6-[2-methyl-3-[1,3,3,3-tetramethyl-1-[(trimethylsilyl)oxy]disiloxanyl]propyl]phenol, 2-Ethylhexyldimethoxybenzylidendioxoimidazolidinpropionat, 1-(3,4-Dimethoxyphenyl)-4,4-dimethyl-1,3-pentandion, 2-(2'-Hydroxy-5'-methoxyphenyl)benzotriazol und Oxybenzon.

2. Transdermale Zubereitung zur äußerlichen Anwendung gemäß Anspruch 1, wobei das NSAID ausgewählt ist aus der Gruppe, bestehend aus Ketoprofen, Tiaprofensäure, Suprofen, Tolmetin, Carprofen, Benoxaprofen, Piroxicam, Benzydamin, Naproxen, Diclofenac, Ibuprofen, Diflunisal, Azapropazon und pharmazeutisch verträglichen Salzen davon.

## Revendications

1. Préparation transdermique pour usage externe, qui contient un NSAID photosensible et un agent bloquant les UV migrant dans la peau, tandis que l'agent bloquant les UV est choisi dans le groupe consistant en 4-tert-butyl-4'-méthoxydibenzoylméthane, n-hexyl 2-(4-diéthylamino-2-hydroxybenzoyl)benzoate, acide 4-hydroxy-3-méthoxycinnamique, un ester d'alkyle ramifié d'acide 4-hydroxy-3-méthoxycinnamique, acide téréphtalylidène-3,3'dicamphor-10,10'-disulfonique, 2-(2H-benzotriazole-2-yl)-4-méthyl-6-[2-méthyl-3-[1,3,3,3-tétraméthyl-1-[(triméthylsilyl)oxy]disiloxanyl]propyl]phénol, 2-éthylhexyl diméthoxybenzylidènedioxoimidazolidinepropionate, 1-(3,4-diméthoxyphényl)-4,4-diméthyl-1,3-pentadione, 2-(2'-hydroxy-5'-méthoxyphényl)-benzotriazole, et oxybenzone.

2. Préparation transdermique pour usage externe selon la revendication 1, dans laquelle le NSAID est choisi dans le groupe consistant en ketoprofen, acide tiaprofénique, suprofen, tolmetin, carprofen, benoxaprofen, piroxicam, benzydamine, naproxen, diclofenac, ibuprofen, diflunisal, azapropazone, et leurs sels pharmaceutiquement acceptables.
